# EUROPEAN PATENT APPLICATION

(11) **EP 4 789 715 A2**
(43) Date of publication of application: **12.08.2026**
(21) Application number: 26169875.7
(22) Date of filing: 14.02.2020
(51) Int. Cl.: A61P 7/04

(54) **METHODS FOR TREATING NEOINTIMAL HYPERPLASIA USING F11R/JAM-A INHIBITORS**

(30) Priority: 19.06.2019 US 201962863477 P
(62) Document number(s) of the earlier application(s) in accordance with Art. 76 EPC: 20826396.2 / 3 986 555
(71) Applicant: The Research Foundation for The State University of New York, Albany, NY 12207 (US)
(72) Inventor: SALIFU, Moro O., New York, 11203 (US); BABINSKA, Anna, New York, 11203 (US)
(74) Representative: Dehns

(57) **Abstract**

The present disclosure relates to methods of treating neointimal hyperplasia. The methods include selecting a subject having a blood vessel abnormality, and administering to the subject an amount of a composition, wherein the composition comprises an inhibitor of cell adhesion molecule receptor/Junctional Adhesion Molecule-A (F11R/JAM-A).

## Description

This application claims benefit of U.S. Provisional Patent Application Serial No. 62/863,477, filed June 19, 2019, which is hereby incorporated by reference in its entirety.

### FIELD

The present disclosure relates generally to methods for treating neointimal hyperplasia using F11R/JAM-A inhibitors.

### BACKGROUND

Neointimal hyperplasia is the proliferation and migration of smooth muscle cells from the media into the intima in response to vessel injury resulting in luminal narrowing. It is initiated by arterial wall damage induced mechanically and/or by shear stress resulting from high flow, pressure and/or turbulence.

The damaged endothelium exposes ligands that attract platelets and recruitment of inflammatory cells and releases of cytokines that facilitate vascular smooth muscle cell proliferation and migration. Clinically, neointimal hyperplasia may be the primary underlying lesion in vaso-oclusive diseases such as stenosis observed in artero-venous fistulas and artero-venous grafts.

Stenosis from neointimal hyperplasia is difficult to treat. Unlike soft atheromatous plaques, stenoses from neointimal hyperplasia are firm and require prolonged high inflation pressures to dilate with a balloon. The stenoses often recur and repeated dilatation causes repeated intimal injury which perpetuates the neointimal hyperplasia.

A stent may be inserted to hold the stenosis open, however, such an approach presents drawbacks. For example, the stent itself will stimulate further neointimal hyperplasia. The intimal tissue may grow through the interstices of a bare stent and re-stenose the vessel. A covered stent may prevent this from happening but neointimal hyperplasia can still occur at the ends of the stent where there is heavy irritation of the vessel wall.

There remains a need for effective methods of treating neointimal hyperplasia. Strategies are under investigation to reduce neointimal hyperplasia such as coating of angioplasty balloons, drug-eluting stents and intravascular brachytherapy, however anticoagulant therapy such as systemic low-dose low molecular weight heparin, and systemic low-dose warfarin or antiplatelet therapies have failed to reduce occurrence of neointimal hyperplasia.

There remains a need to improve methods of treating neointimal hyperplasia. The present disclosure is directed to overcoming these and other deficiencies in the art.

### SUMMARY

A first aspect relates to a method of treating neointimal hyperplasia. The method includes selecting a subject having a blood vessel abnormality, and administering to the subject an amount of a composition, wherein the composition comprises an inhibitor of cell adhesion molecule receptor/Junctional Adhesion Molecule-A (F11R/JAM-A).

### BRIEF DESCRIPTION OF THE DRAWINGS

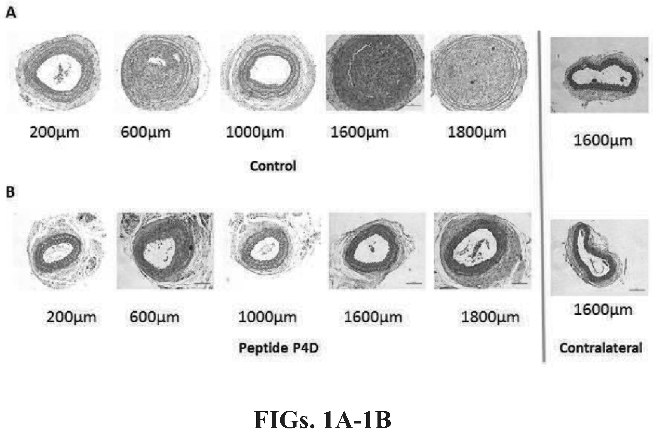

FIGs. 1A-1B show H&E-stained images of cross section of the carotid artery at different distance from the point of ligation, showing that the arterial lumen is preserved in the mice treated with peptide compared to occluded lumens in the controls.

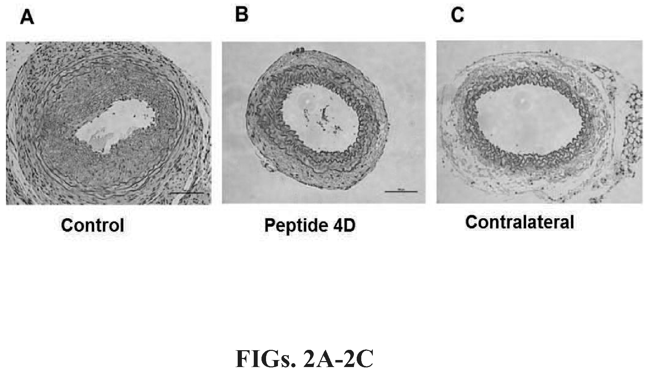

FIGs. 2A-2C depict trichrome-stained images, showing that the lumen of the study artery was preserved, similar to that in the contralateral artery in treated mice and significantly wider than control study artery in untreated mice.

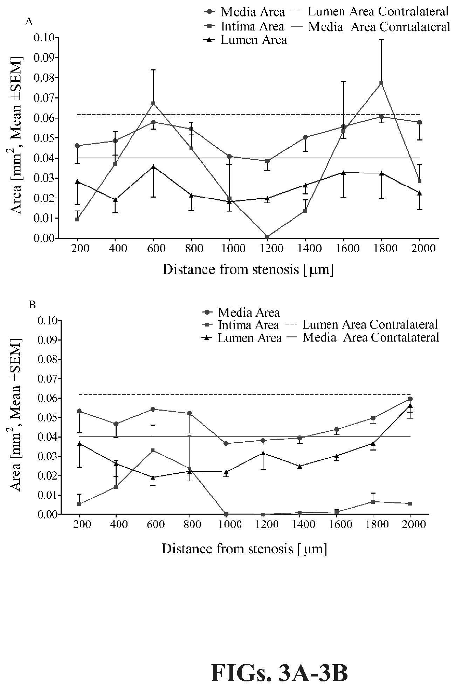

FIGs. 3A-3B are graphical illustrations of mid focal stenosis morphometry of control uremic mice (as shown in FIG. 3A) and uremic mice treated with peptide 4D (as shown in FIG. 3B), showing significantly decreased intima area in the treated mice.

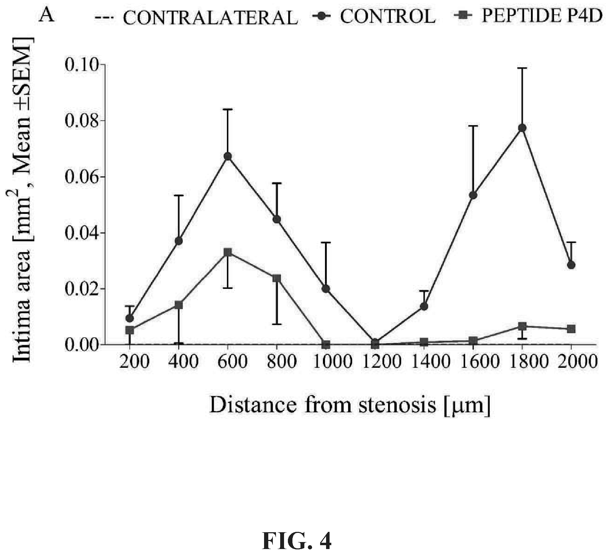

FIG. 4 is graphical illustration of the comparison of mid focal stenosis morphometry in left carotid artery of uremic mice treated with peptide 4D versus control untreated uremic mice, showing that intima area was significantly smaller in treated mice than in control mice at any distance from the ligation point.

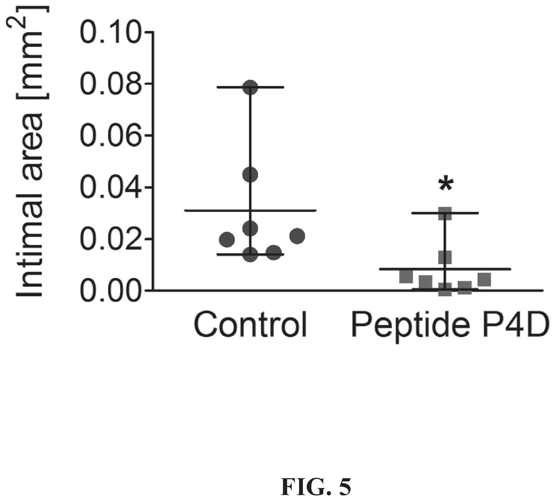

FIG. 5 is a graphical illustration of the comparison of the average of intimal area as a sum of 10 LCA locations with distal focal stenosis of untreated control uremic mice with uremic mice treated with peptide 4D. Peptide 4D treated mice had significantly less mean intima area compared to untreated controls.

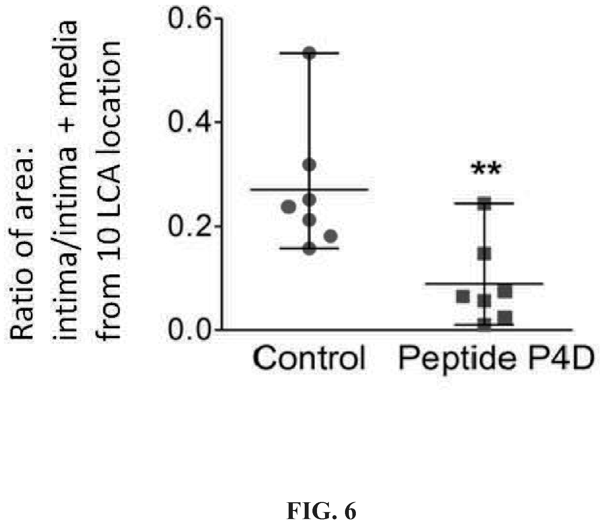

FIG. 6 is a graphical illustration of the comparison of the average of ratio of intima area/intima + media area (Intima Index) from 10 LCA locations with mid focal stenosis of untreated control uremic mice with uremic mice treated with peptide 4D. Peptide 4D treated mice had significantly less mean intima/intima + media ratio compared with controls.

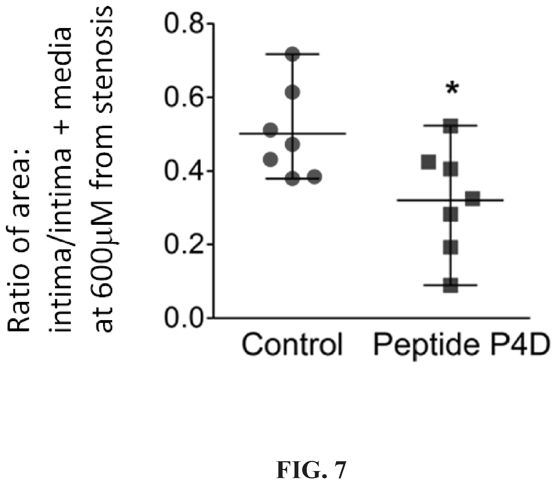

FIG. 7 is a graphical illustration of the comparison of the average of ratio of intima area/intima + media area (Intima Index) from 1 LCA location with biggest distal focal stenosis of untreated control uremic mice with uremic mice treated with peptide 4D. Peptide 4D treated mice had significantly less intima/intima + media ratio compared with controls.

### DETAILED DESCRIPTION

A first aspect relates to a method of treating neointimal hyperplasia. The method includes selecting a subject having a blood vessel abnormality, and administering to the subject an amount of a composition, wherein the composition comprises an inhibitor of cell adhesion molecule receptor/Junctional Adhesion Molecule-A (F11R/JAM-A).

It is to be appreciated that certain aspects, modes, embodiments, variations and features of the present disclosure are described below in various levels of detail in order to provide a substantial understanding of the present technology. The definitions of certain terms as used in this specification are provided below. Unless defined otherwise, all technical and scientific terms used herein generally have the same meaning as commonly understood by one of ordinary skill in the art to which this disclosure belongs.

As used herein, the term "about" means that the numerical value is approximate and small variations would not significantly affect the practice of the disclosed embodiments. Where a numerical limitation is used, unless indicated otherwise by the context, "about" means the numerical value can vary by ±1 or ±10% , or any point therein, and remain within the scope of the disclosed embodiments.

As used herein, the terms "subject," "individual" or "patient," used interchangeably, means any animal, including mammals, such as mice, rats, other rodents, rabbits, dogs, cats, swine, cattle, sheep, horses, or primates, such as humans.

As used herein, the term "purified" means that when isolated, the isolate contains at least 90%, at least 95%, at least 98%, or at least 99% of a compound described herein by weight of the isolate.

As used herein, the phrase "substantially isolated" means a compound that is at least partially or substantially separated from the environment in which it is formed or detected.

It is further appreciated that certain features described herein, which are, for clarity, described in the context of separate embodiments, can also be provided in combination in a single embodiment. Conversely, various features which are, for brevity, described in the context of a single embodiment, can also be provided separately or in any suitable subcombination.

As described herein, neointimal hyperplasia (NIH) (used interchangeably with intimal hyperplasia (IH)) refers to a complex process of smooth muscle cell proliferation and migration from the media into the intima, along with apoptosis and matrix deposition. NIH, as described herein, is a physiological response to injury to a wall of a blood vessel. Endothelial cells express growth factors that promote smooth muscle cells migration from the vessel media to the vessel intima. The smooth muscle cells may then proliferate in the intima and deposit extracellular matrix, in a process analogous to scar formation. In one embodiment, this process occurs over a period of a few months. A healing response leads to thickening (e.g., neointimal hyperplasia) that enters the lumen of the vessel and may cause stenosis. Neointimal hyperplasia in accordance with this disclosure may result from endovascular intervention, vascular surgical procedures, long term vascular catheters and devices, or turbulent flow. Methods of treating atherosclerosis that are known in the art are not considered applicable for treating NIH given the unique mechanism of NIH. *See, e.g.*, Schiener et al., "Nanomedicine-Based Strategies for Treatment of Atherosclerosis," Trends in Molecular Medicine 20(5):271-81 (2014), which is hereby incorporated by reference in its entirety.

In accordance with this aspect of the disclosure, suitable F11R proteins include those derived from a human ortholog of the murine protein called junctional adhesion molecule (JAM), specifically named JAM-1 and JAM-A. "F11R" refers to a receptor protein on the surface of human platelets as a target for a stimulatory M.Ab.F11. "F11R" is also referred to as a human ortholog of the murine protein called junctional adhesion molecule (JAM), specifically named JAM-1 and JAM-A. F11R from either platelets or endothelial cells comprises an extracellular domain consisting of two Ig-folds, a transmembrane domain and a short cytoplasmic portion. The cDNA encoding the F11R can be engineered, e.g., to delete the transmembrane and cytoplasmic domain thereby providing a polynucleotide encoding the extracellular domain. Expression of the F11R extracellular domain in eukaryotic cells results in its synthesis and secretion, thereby indicating that it is a soluble polypeptide.

In one embodiment of the disclosure, the composition comprises an isolated F11R/JAM-A inhibitor. An inhibitor of F11R/JAM-A as described herein may include a peptide that inhibits, suppresses, or causes the cessation of at least one F11R/JAM-A mediated biological activity. In accordance with this aspect of the disclosure, suitable inhibitors of F11R/JAM-A may include protein or peptide inhibitors, nucleic acid inhibitors, or small molecule inhibitors. In one embodiment, the composition comprises an isolated F11R/JAM-A protein or polypeptide. Suitable F11R/JAM-A inhibitors may, for example, include antibodies or antibody fragments recognizing an epitope in the amino acid sequence of F11R/JAM-A.

A peptide as described herein includes a linear series of amino acid residues linked to one another by peptide bonds between the alpha-amino and carboxy groups of adjacent amino acid residues. The term synthetic peptide is intended to refer to a chemically derived chain of amino acid residues linked together by peptide bonds. Synthetic peptide is also intended to refer to recombinantly produced peptides in accordance with the present disclosure. According to the present disclosure, preferred F11R/JAM-A antagonists include peptides (also referred to herein as "F11R antagonist peptides") and antibodies. Additionally, analogs, homologs and fragments of the novel peptides provided herein are included within the scope of the term "F11R/JAM-A antagonist peptide".

"F11R/JAM-A antagonists" and "F11R/JAM-A antagonist peptides" further include any compound that can bind to the active site of the F11R/JAM-A protein, specifically, but not limited to a pocket formed by the N-terminal 23 amino acid region and 13 amino acid region in the first Ig fold. By such binding, the action of F11R/JAM-A is inhibited, i.e., alignment of platelets and endothelial cells in F11R/JAM-A-mediated trans-homophilic interaction through the steric pocket of F11R, is blocked so that platelet aggregation or thrombosis, atherosclerosis, heart attacks, strokes, and all other human disorders that involve thrombus formation, can be prevented or treated. By "F11R/JAM-A antagonist peptide" is meant a peptide that inhibits, suppresses or causes the cessation of at least one F11R/JAM-A mediated biological activity by, for example, interfering with or otherwise preventing the interaction of or binding of F11R/JAM-A to its target (for example, platelets to endothelial cells and thereby inhibit platelet aggregation or interfering with the role of some protein in angiogenesis and thus preventing the growth of tumors). For example F11R/JAM-A antagonist in accordance with the disclosure may include one that interferes with or blocks F11R/JAM-A on another cell, or another protein that F11R/JAM-A binds to such as itself or other JAMs, to the leukocyte function associated antigen-1 (LFA-1) (as described in Ostermann et al., "JAM-1 is a Ligand of the Beta(2) Integrin LFA-1 Involved in Transendothelial Migration of Leukocytes," Nat. Immunol. 3:151-58 (2002), which is hereby incorporated by reference in its entirety), the integrins GPIIb/IIIa and αᵥβ₃, and/or other binding proteins.

F11R/JAM-A inhibitors of the present disclosure may include peptides derived from or corresponding to the F11R/JAM-A which have been isolated and synthesized. These peptides may possess F11R/JAM-A antagonistic properties including the ability to selectively bind to F11R/JAM-A and inhibit F11R/JAM-A-mediated biological activity which, for example, is associated with adhesion of platelets to endothelial cells in mammals. The peptides of the present disclosure may correspond to specific portions of the native human F11 receptor and include variations thereof, and, therefore, are non-immunogenic when administered to humans. The peptides may effectively block collagen-induced platelet aggregation and secretion and thereby are efficacious in regard to, *inter alia,* the prevention of excessive bleeding following an injury, under physiological conditions. Moreover, under pathological conditions, the uncontrolled proliferation and migration of smooth muscle cells into the tunica intima, at the luminal surface of the inflamed endothelium or at exposed collagen sites within the injured vasculature results in excessive smooth muscle cell proliferation and migration to the tunica intima. In the context of the disclosure, the term "subject in need thereof" can be an animal, mammal or human that is at risk for, or is already experiencing symptoms of the foregoing conditions. The ability of the F11R/JAM-A inhibitor to prevent proliferation and migration of smooth muscle cells into the tunica intima ability provides heretofore unrecognized treatment and prevention options in subjects in need thereof, i.e., diseases and disorders associated with proliferation and migration of smooth muscle cells in the tunica intima.

The F11R/JAM-A peptides of the present disclosure substantially correspond to the amino acids of the N-terminus or first Ig domain of human F11R/JAM-A. In one embodiment, the F11R/JAM-A peptide of the present disclosure is a sequence of the N-terminal peptide of the F11R/JAM-A structure: SVTVHSSEPEVRIPENNPVKLSC (SEQ ID NO: 11).

In one embodiment, the F11R/JAM-A peptide of the present disclosure is a sequence within the first Ig fold of the F11R/JAM-A structure: KSVTREDTGTYTC (SEQ ID NO: 2).

As used herein, the F11R/JAM-A inhibitor may include a F11R/JAM-A peptide referred to herein as "P4D" or "peptide 4D" (also referred to herein as F11R/JAM-A- blocking peptide 4D). P4D is a D-amino acid analog of the native peptide, designed for enhanced stability in *in vivo* environment that is abundant in proteases. *See* Babinska et al., "Development of New Antiatherosclerotic and Antithrombotic Drugs Utilizing F11 Receptor (F11R/JAM-A) Peptides," Peptide Science 102(4):322-334 (2014), which is hereby incorporated by reference in its entirety. Therefore, the amino acid sequences of the JAM-A derived peptides that may be useful in this disclosure include, for example, NH₂-(dK)-SVT-(dR)-EDTGTYTC-CONH₂ (SEQ ID NO: 3) for P4D. In one embodiment, a peptidomimetic 4D may be used. Peptidomimetic 4D is a modification of the amino acid sequence of the peptide of SEQ ID NO: 2, resulting in the amino acid sequence of the peptide of SEQ ID NO: 3.

Accordingly, in one embodiment of the present disclosure, the F11R/JAM-A inhibitor comprises an amino acid sequence of SEQ ID NO: 1, an amino acid sequence of SEQ ID NO: 2, an amino acid sequence of SEQ ID NO: 3, an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO: 8, an amino acid sequence of SEQ ID NO: 9, an amino acid sequence of SEQ ID NO: 10, an amino acid sequence of SEQ ID NO: 11, an amino acid sequence of SEQ ID NO: 12, an amino acid sequence of SEQ ID NO: 13, an amino acid sequence of SEQ ID NO: 14, an amino acid sequence of SEQ ID NO: 15, an amino acid sequence of SEQ ID NO: 16, or an amino acid sequence of SEQ ID NO: 17:

**Table 1: Sequences**

| SEQ ID NO | AMINO ACID SEQUENCE | FEATURES |
|---|---|---|
| 1 | XSVTXEDTGTYTC | X₁ is K or dK; X₅ is R or dR; Optional C-terminal Amidation |
| 2 | KSVTREDTGTYTC | |
| 3 | XSVTXEDTGTYTC | X₁ is dK; X₅ is dR; C-terminal Amidation |
| 4 | KSVTREDTGTYTC | C-terminal Amidation |
| 5 | KSVTXEDTGTYTC | X₅ is dR; C-terminal Amidation |
| 6 | XSVTREDTGTYTC | X₁ is dK; C-terminal Amidation |
| 7 | KSVTXEDTGTYTC | X₅ is dR |
| 8 | XSVTREDTGTYTC | X₁ dK |
| 9 | XSVTXEDTGTYTC | X₁ is dK; X₅ is dR |
| 10 | SVTVHSSEPEVXIPENNPVKLSC | X₁₂ is R or dR; Optional C-terminal Amidation |
| 11 | SVTVHSSEPEVXIPENNPVKLSC | |
| 12 | SVTVHSSEPEVRIPENNPVKLSC | C-terminal Amidation |
| 13 | SVTVHSSEPEVXIPENNPVKLSC | X₁₂ dR |
| 14 | SVTVHSSEPEVXIPENNPVKLSC | X₁₂ is dR; C-terminal Amidation |
| 15 | SSEPEVXIPENNPV | X₇ is R or dR |
| 16 | SSEPEVRIPENNPV | |
| 17 | SSEPEVXIPENNPV | X₇ is dR |

In another embodiment of the present disclosure, the F11R/JAM-A inhibitor comprises an amino acid sequence having about 70-80% sequence similarity to any of SEQ ID NOs: 1-17, more preferably, about 80-90% sequence similarity to any of SEQ ID NOs: 1-17, and more preferably 90-95% sequence similarity to any of SEQ ID NOs: 1-17, and most preferably about 95-99% sequence similarity to any of SEQ ID NOs: 1-17. In a preferred embodiment, the peptide is an amino acid sequence of SEQ ID NO: 2.

Thus, unless indicated to the contrary, both the immature and the mature forms of native F11R/JAM-A, and the sequences having less than 100% similarity with native F11R/JAM-A (i.e., native sequences and analogs alike, collectively referred to herein as "F11R/JAM-A") may be used in the methods of the present disclosure.

The present disclosure is also directed to homologs, analogs, and fragments of these peptides which maintain F11R/JAM-A- antagonist activity in a subject, particularly mammals and more particularly humans, are also contemplated by the present disclosure.

F11R/JAM-A proteins and polypeptides of the disclosure may differ from the native polypeptides, in terms of one or more additional amino acid insertions, substitutions or deletions, e.g., one or more amino acid residues within SEQ ID NOs: 1-17 may be substituted by another amino acid of a similar polarity, which acts as a functional equivalent, resulting in a silent alteration. That is to say, the change relative to the native sequence would not appreciably diminish the basic properties of native F11R/JAM-A. Any such analog of F11R/JAM-A may be screened to determine if it maintains native F11R/JAM-A activity. Substitutions within these proteins and polypeptides may be selected from other members of the class to which the amino acid belongs. For example, nonpolar (hydrophobic) amino acids include glycine, alanine, leucine, isoleucine, valine, proline, phenylalanine, tryptophan and methionine. Polar neutral amino acids include serine, threonine, cysteine, tyrosine, asparagine, and glutamine. Positively charged (basic) amino acids include arginine, lysine and histidine. Negatively charged (acidic) amino acids include aspartic acid and glutamic acid.

As used herein a "mimetic" or "peptidomimetic" of a compound's functional site refers to a compound in which chemical structures of protein or peptide sequences necessary for functional activity of a compound's functional site have been replaced with other chemical structures that mimic the conformation of the functional site. An example of a peptidomimetic contemplated by the present disclosure includes a compound (e.g., a small organic molecule) including portions with residues which interact sterically with the binding site of the F11R molecule. In accordance with the present disclosure, F11R peptidomimetic drugs can be designed on the basis of, for example, peptides having SEQ ID NOs: 11 or 2 and the tertiary structure of the binding site of F11R, including parts of the protein containing these sequences. Such peptidomimetic drugs with structural relationships analogous to that observed between morphine, enkephalins and beta-endorphins, are suitable as therapeutic agents. The design and synthesis of peptidomimetic molecules continues to be at the forefront of drug design and discovery and many peptidomimetic frameworks and methods for their synthesis have been developed (Babine & Bender, "Molecular Recognition of Proteinminus signLigand Complexes: Applications to Drug Design," Chem. Rev. 97:1359-1472 (1997); Hanessian et al., "Design and Synthesis of Conformationally Constrained Amino Acids as Versatile Scaffolds and Peptide Mimetics," Tetrahedron, 53:12789-854 (1997); Fletcher & Campbell, "Partially Modified Retro-Inverso Peptides: Development, Synthesis, and Conformational Behavior," Chem. Rev., 98:763-96 (1998), all of which are hereby incorporated by reference in their entirety).

The peptidomimetics in accordance with the present disclosure can be developed, for example, with the aid of computerized molecular modeling. In one embodiment, the present disclosure provides a composition comprising SEQ ID NO: 11 or SEQ ID NO: 2 wherein SEQ ID NO: 11 or SEQ ID NO: 2 comprises peptidomimetics that are capable of specific binding with the F11R binding site. Peptide mimetics that are structurally similar to therapeutically useful peptides can be used to produce an equivalent therapeutic or prophylactic effect. Generally, peptidomimetics are structurally similar to a paradigm polypeptide (i.e., a polypeptide that has a biochemical property or pharmacological activity), such as SEQ ID NO: 11 or SEQ ID NO: 2, but have one or more peptide linkages optionally replaced by a linkage selected from the group consisting of: -CH₂NH-, -CH₂S-, -CH₂CH₂-, -CH=CH- (cis and trans), - COCH₂-, -CH(OH)CH₂-, and -CH₂SO-, by methods known in the art and further described in the following references: Spatola, A. F. in CHEMISTRY AND BIOCHEMISTRY OF AMINO ACIDS, PEPTIDES, AND PROTEINS (B. Weinstein, eds., Marcel Dekker, p. 267, 1983); Hudson et al., "Methionine Enkephalin and Isosteric Analogues. I. Synthesis on a Phenolic Resin Support," Int. J. Pept. Prot. Res. 14:177-185 (1979) (-CH₂NH-, -CH₂-CH₂-); Spatola et al., "Structure-activity Relationships of Enkephalins Containing Serially Replaced Thiomethylene Amide Bond Surrogates," Life Sci. 38:1243-1249 (1986) (-CH₂S); Hann et al., "On the Double Bond Isostere of the Peptide Bond: Preparation of an Enkephalin Analogue," Chem. Soc. Perkin Trans. I 307-314 (1982) (-CH=CH-, cis and trans); Almquist et al., "Synthesis and Biological Activity of a Ketomethylene Analogue of a Tripeptide Inhibitor of Angiotensin Converting Enzyme," J. Med. Chem. 23:1392-98 (1980) (-COCH₂-); Jennings-White et al., "Synthesis of Ketomethylene Analogs of Dipeptides," Tetrahedron Lett 23:2533-34 (1982) (-COCH₂-); EP0045665 (-CH(OH)CH₂-); Holladay et al., "Synthesis of Hydroxyethylene and Ketomethylene Dipeptide Isosteres," Tetrahedron Lett 24:4401-04 (1983) (-C(OH)CH₂-); and Hruby, V. J., "Conformational Restrictions of Biologically Active Peptides via Amino Acid Side Chain Groups," Life Sci. 31:189-99 (1982) (-CH₂S-), all of which are hereby incorporated by reference in their entirety.

In another embodiment, a non-peptide linkage is -CH₂NH-. Such peptidomimetics may have significant advantages over polypeptide embodiments, including, for example: more economical production, greater chemical stability, enhanced pharmacological properties (half-life, absorption, potency, efficacy, etc.), altered specificity (e.g., a broad-spectrum of biological activities), reduced antigenicity, and others.

A variety of designs for peptidomimetics are possible. For example, cyclic peptides, in which the necessary conformation for binding is stabilized by nonpeptides, are specifically contemplated. U.S. Pat. Nos. 5,192,746 to Lobl et al.; 5,169,862 to Burke, Jr. et al.; 5,539,085 to Bischoff et al.; 5,576,423 to Aversa et al.; 5,051,448 to Shashoua; and 5,559,103 to Gaeta et al., all of which are hereby incorporated by reference in their entirety, describe multiple methods for creating such compounds. Synthesis of nonpeptide compounds that mimic peptide sequences is also known in the art. Eldred et al., "Orally Active Non-Peptide Fibrinogen Receptor (GpIIb/IIIa) Antagonists: Identification of 4-[4-[4-(aminoiminomethyl)phenyl]-1-piperazinyl]-1-piperidineacetic Acid as a Long-Acting, Broad-Spectrum Antithrombotic Agent," J. Med. Chem. 37:3882-85 (1994), which is hereby incorporated by reference in its entirety, describes nonpeptide antagonists that mimic the peptide sequence. Likewise, Ku et al., "Potent Non-Peptide Fibrinogen Receptor Antagonists Which Present an Alternative Pharmacophore," J. Med. Chem. 38:9-12 (1995), which is hereby incorporated by reference in its entirety give further elucidation of the synthesis of a series of such compounds. Derivatives of e.g. SEQ ID NO: 11 or SEQ ID NO: 2 can be produced using recombinant nucleic acid molecule techniques.

Modifications to a specific peptide may be deliberate, as through site-directed mutagenesis and amino acid substitution during biosynthesis, or may be accidental such as through mutations in hosts, which produce the peptide. Peptides including derivatives can be obtained using standard mutagenesis techniques such as those described in Sambrook et al., MOLECULAR CLONING, Cold Spring Harbor Laboratory Press (1989), which is hereby incorporated by reference in its entirety. For example, Chapter 15 of Sambrook describes procedures for site-directed mutagenesis of cloned DNA. Derivatives of SEQ ID NOs.: 11 and 2 include, but are not limited by modification occurring during or after translation, for example, by phosphorylation, glycosylation, crosslinking, acylation, proteolytic cleavage, linkage to a therapeutic protein, an antibody molecule, membrane molecule or other ligand (see Ferguson et al., "Cell-surface Anchoring of Proteins via Glycosyl-Phosphatidylinositol Structures," Annu. Rev. Biochem. 57:285-320 (1988), which is hereby incorporated by reference in its entirety). Specific types of genetically produced derivatives also include, but not limited by amino acid alterations such as deletions, substitutions, additions, and amino acid modifications. A deletion refers to the absence of one or more amino acid residue(s) in the related peptide. An addition refers to the presence of one or more amino acid residue(s) in the related peptide. Additions and deletions to a peptide may be at the amino terminus, the carboxy terminus, and/or internal, can be produced by mutation in e.g., DNA encoding SEQ ID NOs: 11 or 2, and/or by peptide posttranslation modification. Amino acid modification refers to the alteration of a naturally occurring amino acid to produce a non-naturally occurring amino acid. Analogs of, for example, SEQ ID NOs: 11 or 2 with unnatural amino acids can be created by site-specific incorporation of unnatural amino acids into polypeptides during the biosynthesis, as described in Noren et al., "A General Method for Site-Specific Incorporation of Unnatural Amino Acids Into Proteins," Science 244:182-88 (1989), which is hereby incorporated by reference in its entirety. A substitution refers to the replacement of one or more amino acid residue(s) by another amino acid residue(s) in the peptide. Mutations can be made in, for example, DNA encoding SEQ ID NOs: 11 or 2 such that a particular codon is changed to a codon, which codes for a different amino acid. Such a mutation is generally made by making the fewest nucleotide changes possible. A substitution mutation of this sort can be made to change an amino acid in the resulting peptide in a non-conservative manner (i.e., by changing the codon from an amino acid belonging to a grouping of amino acids having a particular size or characteristic to an amino acid belonging to another grouping) or in a conservative manner (i.e., by changing the codon from an amino acid belonging to a grouping of amino acids having a particular size or characteristic to an amino acid belonging to the same grouping). Such a conservative change generally leads to less change in the structure and function of the resulting peptide. To some extent the following groups contain amino acids which are interchangeable: the basic amino acids lysine, arginine, and histidine; the acidic amino acids aspartic and glutamic acids; the neutral polar amino acids serine, threonine, cysteine, glutamine, asparagine and, to a lesser extent, methionine; the nonpolar aliphatic amino acids glycine, alanine, valine, isoleucine, and leucine (however, because of size, glycine and alanine are more closely related and valine, isoleucine and leucine are more closely related); and the aromatic amino acids phenylalanine, tryptophan, and tyrosine. In addition, although classified in different categories, alanine, glycine, and serine seem to be interchangeable to some extent, and cysteine additionally fits into this group, or may be classified with the polar neutral amino acids. Although proline is a nonpolar neutral amino acid, its replacement represents difficulties because of its effects on conformation. The conformation conferring properties of proline residues may be obtained if one or more of these is substituted by hydroxyproline (Hyp). Derivatives can contain different combinations of alterations including more than one alteration and different types of alterations.

The ability of the derivative to retain some activity can be measured using techniques described herein and/or using techniques known to those skilled in the art for measuring the F11R receptor binding activity. Derivatives of e.g., SEQ ID NOs: 11 or 2 are functional equivalents having similar amino acid sequence and retaining, to some extent, the activities of SEQ ID NO: 11 or 2. By functional equivalent is meant the derivative has an activity that can be substituted for the activity of SEQ ID NO: 11 or 2. Preferred functional equivalents retain the full level of F11R-binding activity as measured by assays known to these skilled in the art. Preferred functional equivalents have activities that are within 1% to 10,000% of the activity of, for example, SEQ ID NO: 11 or 2, more preferably between 10% to 1000%, and more preferably within 50% to 200%. Derivatives have at least 50% sequence similarity, preferably 70%, more preferably 90%, and even more preferably 95% sequence similarity to SEQ ID NO: 11 or 2. Sequence similarity includes homology observed between amino acid sequences in two different polypeptides, irrespective of polypeptide origin. A residue refers to an amino acid incorporated in the peptide by an amide bond, for example. Approaches to designing peptide mimetics are known in the art. For example, *see* Farmer, P. S. in DRUG DESIGN (E. J. Ariens, ed. Academic Press, 1980), vol. 10, pp. 119-143; Ball & Alewood, "Conformational Constraints: Nonpeptide Beta-Turn Mimics," J. Mol. Recognition 3:55-64 (1990); and Freidinger RM, "Non-Peptide Ligands for Peptide Receptors," Trends Pharmacol. Sci. 10:270-74 (1989), which are hereby incorporated by reference in their entirety. In one embodiment, the present disclosure contemplates all peptidomimetics which can be designed based on the knowledge of the sequence and of the three-dimensional structure of the F11R molecule of the disclosure including but not limited to all mimetic compounds which can be conventionally synthesized by an ordinarily skilled chemist to bind to, antagonize, act as an agonist, inhibit, promote, block, or otherwise functionally interact with the binding site of the F11R.

In one embodiment, compounds of the present methods are peptides which are made to resemble the monoclonal antibody F11 ("M.Ab.F11") binding site on platelets. In one example a peptidomimetic entitled 4D may be used. Peptidomimetic 4D has the amino acid sequence of the peptide of SEQ ID NO: 3, a modification of SEQ ID NO: 2. SEQ ID NO: 2 is a 13 amino acid sequence located within the first immunoglobulin fold of the F11R protein. A 13-mer peptide with this sequence is an inhibitor of the biological activities of F11R measured both *in vitro* and in cell cultures. A non-natural peptide that mimics the structure of the peptide of SEQ ID NO: 2 can be synthesized with substituted D-amino acids in either of the two main sites for cleavage by endopeptidases trypsin and optionally with the carboxyl group amidated (CONH₂) for the prevention of dimeric-disulphides. An example is SEQ ID NO: 3, wherein Lys corresponding to amino acid 1 of SEQ ID NO: 2 is replaced with D-Lys, Arg corresponding to amino acid 5 of SEQ ID NO: 2 is replaced with D-Arg, and the C-terminal Cys corresponding to the C-terminal Cys of SEQ ID NO: 2 is amidated. Substituted D-amino acids are in the two main sites for cleavage by endopeptidases trypsin and the carboxyl group amidation (CONH₂) may prevent formation of dimeric-disulphides. Other examples include any of the eight possible combinations of the foregoing D-amino acid substitutions and optional C-terminal amidation, as represented by SEQ ID NO: 1 generally, and specifically by SEQ ID NOs: 2-9.

In another embodiment, a peptidomimetic entitled 1P may be used. Peptidomimetic 1P has the amino acid sequence of the peptide of SEQ ID NO: 14, a modification of SEQ ID NO: 11. SEQ ID NO: 11 is a 23-amino acid sequence located within the N-terminus of the FllR protein.. A 23-mer peptide with this sequence is an inhibitor of the biological activities of F11R. A non-natural peptide that mimics the structure of the peptide of SEQ ID NO: 11 can be synthesized with a substituted D-amino acid a main sites for cleavage by endopeptidases and optionally with the carboxyl group amidated (CONH₂) for the prevention of dimeric-disulphides. An example is SEQ ID NO: 14, wherein Arg corresponding to amino acid 12 of SEQ ID NO: 11 is replaced with D- Arg and the C-terminal Cys corresponding to the C-terminal Cys of SEQ ID NO: 2 is amidated. Substituted D-amino acid. Other examples include any of the four possible combinations of of the foregoing D-amino acid substitution and optional C-terminal amidation, as represented by SEQ ID NO: 10 generally, and specifically by SEQ ID NOs: 11-14.

In another embodiment, a peptidomimetic entitled 1D may be used. Peptidomimetic 1D is 14-amino acid fragment corresponding to amino acids 6-19 of SEQ ID NO: 11, and is represented by SEQ ID NO: 16. A 14-mer peptide with this sequence is an inhibitor of the biological activities of F11R. A non-natural peptide that mimics the structure of the peptide of SEQ ID NO: 16 can be synthesized with a substituted D-amino acid a main site for cleavage by endopeptidase. An example is SEQ ID NO: 17, wherein Arg corresponding to amino acid 7 of SEQ ID NO: 15 is replaced with D- Arg. Other examples include any of the two possible combinations of the foregoing D-amino acid substitution, as represented by SEQ ID NO: 15 generally, and specifically by SEQ ID NOs: 16 and 17.

All of the foregoing sequences possess anti F11R-activity and as such can be used interchangeably or in combination with any additional one or more of each other for uses disclosed herein, including treating neointimal hyperplasia by selecting a subject having a blood vessel abnormality, and administering to said subject an amount of a composition including a peptide having an amino acid sequence of any one of SEQ ID NOs: 1-17. In an example, the peptide is selected from any one or more of an amino acid sequence of SEQ ID NO: 1, an amino acid sequence of SEQ ID NO: 2, an amino acid sequence of SEQ ID NO: 3, an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO: 8, an amino acid sequence of SEQ ID NO: 9, an amino acid sequence of SEQ ID NO: 10, an amino acid sequence of SEQ ID NO: 11, an amino acid sequence of SEQ ID NO: 12, an amino acid sequence of SEQ ID NO: 13, an amino acid sequence of SEQ ID NO: 14, and an amino acid sequence of SEQ ID NO: 15, an amino acid sequence of SEQ ID NO: 16, and an amino acid sequence of SEQ ID NO: 17.

In an example, the peptide is selected from an amino acid sequence of SEQ ID NO: 1, an amino acid sequence of SEQ ID NO: 2, an amino acid sequence of SEQ ID NO: 3, an amino acid sequence of SEQ ID NO: 10, an amino acid sequence of SEQ ID NO: 11, an amino acid sequence of SEQ ID NO: 14, an amino acid sequence of SEQ ID NO: 15, an amino acid sequence of SEQ ID NO: 16, or an amino acid sequence of SEQ ID NO: 17. In an example, the peptide is an amino acid sequence of SEQ ID NO: 3. In an example, the peptide is an amino acid sequence of SEQ ID NO: 14. In an example, the peptide is an amino acid sequence of SEQ ID NO: 17.

In an example, the blood vessel abnormality is an arterio-venous fistula or an arterio-venous graft. In another example, the subject has a condition, and said condition is selected from the group consisting of chronic kidney disease and end stage renal disease. In another example, the subject has undergone a procedure, and said procedure is selected from the group consisting of surgical revision and angioplasty. In another example, the subject has undergone renal replacement therapy. In an example, the renal replacement therapy is hemodialysis. In another example the composition further includes a pharmaceutically acceptable excipient. In another example, administration is carried out parenterally. In another example, administration is carried out by intravenous injection, intra-arterial injection, intramuscular injection, intraperitoneally, topically, transdermally, subcutaneously, orally, or any combination thereof. Another example further includes repeating the administering. In another example the subject is in hemodialysis. In another example the composition includes a polyethylene glycol tag.

The modifications included in SEQ ID NO: 3 resulted in the design of a mimeticpeptide referred to herein as peptide 4D. Examples of peptidomimetic 4D, 1P, 1D, and inhibitory activity of all of SEQ ID NOs: 1-17 are described in Babinska et al., "F11-receptor (F11R/JAM) Mediates Platelet Adhesion to Endothelial Cells: Role in Inflammatory Thrombosis," Thrombosis & Haemostasis 88(5):843-50 (2002), US Patent No. 9,556,235, and US Patent No. 8,557,957, all of which are hereby incorporated by reference in their entireties. As disclosed herein, an inhibitory effect of a composition including one or morepeptides having one or more of the amino acid sequences represented by SEQ ID NOs: 1-17 indicates an effect on treatment of neointimal hyperplasia.

A homolog in accordance with the present disclosure includes the corresponding peptides from F11R/JAM-A proteins of other mammalian species substantially homologous at the overall protein (i.e., mature protein) level to human F11R/JAM-A, so long as such homologous peptides retain the F11R/JAM-A antagonist activity.

An analog in accordance with the present disclosure includes peptides which differ by one or more amino acid alterations, which alterations, e.g., substitutions, additions or deletions of amino acid residues, do not abolish the F11R/JAM-A antagonist properties of the relevant peptides. Thus, an analog can comprise a peptide having a substantially identical amino acid sequence to a peptide provided herein and in which one or more amino acid residues have been conservatively or non-conservatively substituted. Examples of conservative substitutions include the substitution of a non-polar (hydrophobic) residue such as isoleucine, valine, leucine or methionine for another. Likewise, the present disclosure contemplates the substitution of one polar (hydrophilic) residue such as between arginine and lysine, between glutamine and asparagine, and between glycine and serine. Additionally, the substitution of a basic residue such as lysine, arginine or histidine for another or the substitution of one acidic residue such as aspartic acid or glutamic acid for another is also contemplated. Examples of non-conservative substitutions include the substitution of a non-polar (hydrophobic) residue such as isoleucine, valine, leucine, alanine, methionine for a polar (hydrophilic) residues such as cyteine, glutamine, glutamic acid, lysine and/or a polar residue for a non-polar residue.

In other embodiments, non-conservative alterations (e.g., one or amino acid substitutions, deletions and/or additions) can be made to F11R/JAM-A. Molecular alterations can be accomplished by methods well known in the art, including primer extension on a plasmid template using single stranded templates (Kunkel et al., "A Simple and Efficient Method for the Oligodeoxyribonucleotide-Directed Mutagenesis of Double-Stranded Plasmid DNA," Proc. Acad. Sci. USA 82:488-492 (1985), which is hereby incorporated by reference in its entirety), double stranded DNA templates, and by PCR cloning (Braman, J. (ed.), IN VITRO MUTAGENESIS PROTOCOLS, 2nd ed. Humana Press, Totowa, N.J. (2002), which is hereby incorporated by reference in its entirety) or by using pegylation in which polyethylene glycol is used to tag each of the sequences for longer half-life (Veronese et al., "The Impact of PEGylation on Biological Therapies," BioDrugs 22(5):315-29 (2008). In one embodiment, the composition comprises a polyethylene glycol tag.

A conservative substitution as disclosed herein includes the use of chemically derivatized residues in place of a non-derivatized residues as long as the peptide retains the requisite F11R/JAM-A antagonist, inhibition properties as conventionally measured. Analogs also include the presence of additional amino acids or the deletion of one or more amino acids which do not affect F11R/JAM-A-mediated biological activity. For example, analogs of the subject peptides can contain an N- or C-terminal cysteine, by which, if desired, the peptide can be covalently attached to a carrier protein, e.g., albumin. Such attachment, it is believed, will minimize clearing of the peptide from the blood and also prevent proteolysis of the peptides. In addition, for purposes of the present disclosure, peptides containing D-amino acids in place of L-amino acids are also included in the term "conservative substitution." The presence of such D-isomers can help minimize proteolytic activity and clearing of the peptide.

The term fragment as described herein refers to any subject peptide having an amino acid sequence shorter than that of any peptide depicted in the submitted sequence listing and which fragment retains the F11R/JAM-A-mediated antagonist activity of the subject peptides.

In one embodiment of the present disclosure, the F11R/JAM-A inhibitor is a highly purified F11R/JAM-A preparation. Examples of a highly purified F11R/JAM-A preparation include F11R/JAM-A proteins or polypeptides of SEQ ID NOs: 1-17. An isolated protein or polypeptide as described herein refers to a protein or polypeptide that has been separated from other proteins, lipids, and nucleic acids with which it is naturally associated with. Purity may be measured by any appropriate standard method, for example, by column chromatography, polyacrylamide gel electrophoresis, of HPLC analysis. An isolated protein or polypeptide of the disclosure can be purified from a natural source, produced by recombinant DNA techniques, or produced by chemical methods.

The compositions of the present disclosure may be prepared by formulating a F11R/JAM-A inhibitor (e.g., a F11R/JAM-A peptide) with a pharmaceutically acceptable carrier and optionally a pharmaceutically acceptable excipient. In one embodiment, the composition comprises a pharmaceutically acceptable excipient. As used herein, the terms "pharmaceutically acceptable carrier" and "pharmaceutically acceptable excipient" (e.g., additives such as diluents, immunostimulants, adjuvants, antioxidants, preservatives and solubilizing agents) are non-toxic to the cell or mammal being exposed thereto at the dosages and concentrations employed. Examples of pharmaceutically acceptable carriers include water, e.g., buffered with phosphate, citrate, and another organic acid. Representative examples of pharmaceutically acceptable excipients that may be useful in the present disclosure include antioxidants such as ascorbic acid; low molecular weight (less than about 10 residues) polypeptides; proteins, such as serum albumin, gelatin, or immunoglobulins; adjuvants (selected so as to avoid adjuvant-induced toxicity, such as a β-glucan as described in U.S. Patent 6,355,625, which is hereby incorporated by reference in its entirety, or a granulocyte colony stimulating factor (GCSF)); hydrophilic polymers such as polyvinylpyrrolidone; amino acids such as glycine, glutamine, asparagine, arginine, or lysine; monosaccharides, disaccharides, and other carbohydrates including glucose, mannose, or dextrins; chelating agents such as EDTA; sugar alcohols such as mannitol or sorbitol; salt forming counterions such as sodium; and/or nonionic surfactants such as TWEEN^{®}, polyethylene glycol (PEG), and PLURONICS^{®}.

Compositions of the present disclosure may be prepared for storage by mixing the active ingredient(s) having the desired degree of purity with the pharmaceutically acceptable carrier and optional excipient and/or additional active agent, in the form of lyophilized formulations or aqueous solutions.

The practice of the present disclosure employs, unless otherwise indicated, conventional techniques of synthetic organic chemistry, protein chemistry, molecular biology, microbiology, and recombinant DNA technology, which are well within the skill of the art. These techniques are applied in connection with peptide synthesis, recombinant production of peptides and peptide mutagenesis, for example. Such techniques are explained in the literature (*see e.g.*, Scopes, R.K., PROTEIN PURIFICATION: PRINCIPLES AND PRACTICES, 2nd ed. (Springer-Verlag. 1987); METHODS IN ENZYMOLOGY (M. Deutscher, ed., Academic Press, Inc. 1990); Sambrook, J., MOLECULAR CLONING: A LABORATORY MANUAL, 2nd ed. (Cold Spring Harbor Press 1989); HANDBOOK OF EXPERIMENTAL IMMUNOLOGY, Vols. I-IV (D.M. Weir and C.C. Blackwell, eds., Blackwell Scientific Publications 1986); and House, H., MODERN SYNTHETIC REACTIONS, 2d ed., (W.A. Benjamin 1972), all of which are hereby incorporated by reference in their entirety).

The peptides of the present disclosure, homologs, analogs, and fragments thereof may be synthesized by known techniques. For example, the peptides may be prepared using the solid-phase synthetic technique initially described by Merrifield, R.B., "Solid Phase Peptide Synthesis. I. The Synthesis of a Tetrapeptide," J. Am. Chem. Soc. 85:2149-54 (1963), which is hereby incorporated by reference in its entirety.

Other peptide synthesis techniques can be found in Bodanszky, M., PRINCIPLES OF PEPTIDE SYNTHESIS, 2nd ed. (John Wiley & Sons 1976), which is hereby incorporated by reference, and other references readily available to those skilled in the art. A summary of polypeptide synthesis techniques can be found in Stuart J.M. and Young, J.D., SOLID PHASE PEPTIDE SYNTHESIS (Pierce Chemical Company 1984). Peptides may also be synthesized by solution methods as described in THE PROTEINS, Vol. II. 3rd ed. (Neurath, H. et al., Eds., p. 105-237, Academic Press 1976), which is hereby incorporated by reference in its entirety. Appropriate protective groups for use in different peptide syntheses are described in the above-mentioned texts as well as in McOmie, J.F.W., PROTECTIVE GROUPS IN ORGANIC CHEMISTRY (Plenum Press 1973), which is hereby incorporated by reference in its entirety. The peptides of the present disclosure can also be prepared by chemical or enzymatic cleavage from larger portions of the F11R/JAM-A molecule or from the entire F11R/JAM-A molecule.

Additionally, the peptides of the present disclosure may also be prepared by recombinant DNA techniques (*see e.g.*, Ausubel et al., CURRENT PROTOCOLS IN MOLECULAR CLONING (John Wiley & Sons 1995); Sambrook, J., MOLECULAR CLONING: A LABORATORY MANUAL, 2nd ed. (Cold Spring Harbor Press 1989); Coligan et al. CURRENT PROTOCOLS IN IMMUNOLOGY, (John Wiley & Sons Inc. 1994), all of which are hereby incorporated by reference in their entirety). The skilled artisan understands that any of a wide variety of expression systems can be used to provide the recombinant peptides of the present disclosure. The precise host cell used is not critical to the disclosure. The F11R/JAM-A antagonist peptides can be produced in a prokaryotic host (e.g., *E. coli*), or in a eukaryotic host (e.g., *S. cerevisiae*), mammalian cells (e.g., COS1, CHO, NIH3T3, and JEG3 cells), or in the cells of an arthropod (e.g. *S. frugiperda*). Such cells are available from e.g. the American Type Culture Collection, Manassas, VA. The method of transfection and the choice of expression vehicle will depend on the host system selected. Transformation and transfection methods are described, e.g. in Sambrook, J., MOLECULAR CLONING: A LABORATORY MANUAL, 2nd ed. (Cold Spring Harbor Press 1989), which is incorporated by reference in its entirety; expression vehicles can be chosen from those provided e.g. in Powels et al., CLONING VECTORS: A LABORATORY MANUAL (1985), Supp. 1987, which is hereby incorporated by reference in its entirety.

For most of the amino acids used to build proteins, more than one coding nucleotide triplet (codon) can code for a particular amino acid residue. This property of the genetic code is known as redundancy. Therefore, a number of different nucleotide sequences can code for a particular subject F11R/JAM-A antagonist peptide. The present disclosure also contemplates a deoxyribonucleic acid (DNA) molecule or segment that defines a gene coding for, i.e., capable of expressing, a subject peptide or a subject chimeric peptide from which a peptide of the present disclosure can be enzymatically or chemically cleaved.

DNA molecules that encode peptides of the present disclosure can be synthesized by chemical techniques, for example, the phosphotriester method of Matteucci et al., "Synthesis of Deoxyoligonucleotides on a Polymer Support," J. Am. Chem. Soc. 103:3185 (1981), which is hereby incorporated by reference in its entirety. Using a chemical DNA synthesis technique, desired modifications in the peptide sequence can be made by making substitutions for bases which code for the native amino acid sequence. Ribonucleic acid equivalents of the above described DNA molecules may also be used.

A nucleic acid molecule comprising a vector capable of replication and expression of a DNA molecule defining coding sequence for a subject polypeptide or subject chimeric polypeptide is also contemplated.

The peptides of the present disclosure may be chemically synthesized by conventional techniques such as the Merrifield solid phase technique. In general, the method comprises the sequential addition of one or more amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

A preferred method of solid phase synthesis entails attaching the protected or derivatized amino acid to an inert solid support through its unprotected carboxyl or amino group. The protecting group of the amino or carboxyl group is then selectively removed and the next amino acid in the sequence having the complementary (amino or carboxyl) group suitably protected is admixed and reacted under conditions suitable for forming the amide linkage with the residue already attached to the solid support. The protecting group of the amino carboxyl group is then removed from this newly added amino acid residue, and the next amino acid (suitably protected) is then added, and so forth. After all the desired amino acids have been linked in the proper sequence, any remaining terminal and side group protecting groups including the solid support are removed sequentially or concurrently to yield the final peptide. The lyophilized oligopeptides are resuspended in double distilled H₂O at 2 mg/ml as stock solutions and subsequently diluted in M199-HPS for experiments.

The composition may, in one embodiment, comprise an anti-F11R/JAM-A antibody or antigen-binding fragment thereof. Suitable anti-F11R/JAM-A antibodies, for example, include those antibodies recognizing one or more epitopes in the amino acid sequence of SEQ ID NOs: 1-17. In yet another embodiment, the anti-F11R/JAM-A antibody composition is multivalent in that it also contains an antibody that specifically binds another bacterial antigen (and that optionally neutralizes the other bacterial antigen).

The term antibody as described herein includes monoclonal antibodies, antibody fragments, polyclonal antibodies, genetically engineered forms of the antibodies, and combinations thereof. More specifically, the term antibody, which may be used interchangeably with the term immunoglobulin, includes full length (i.e., naturally occurring or formed by normal immunoglobulin gene fragment recombinatorial processes) immunoglobulin molecules (e.g., an IgG antibody) and immunologically active fragments thereof (i.e., including the specific binding portion of the full-length immunoglobulin molecule), which may be naturally occurring or synthetic in nature. The term antibody fragment includes a portion of an antibody such as F(ab')₂, F(ab)₂, Fab', Fab, Fv, scFv, and the like. Regardless of structure, an antibody fragment binds with the same antigen that is recognized by the full-length antibody, and, in the context of the present disclosure, specifically binds F11R/JAM-A. Methods of making and screening antibody fragments are well-known in the art.

Naturally occurring antibodies generally have two identical heavy chains and two identical light chains, with each light chain covalently linked to a heavy chain by an inter-chain disulfide bond and multiple disulfide bonds further link the two heavy chains to one another. Individual chains may fold into domains that have similar sizes and structures but different functions. The light chain may comprise one variable domain (VL) and/or one constant domain (CL). The heavy chain may comprise one variable domain (VH) and/or, depending on the class or isotype of antibody, three or four constant domains (C_{H}1, C_{H}2, C_{H}3, and C_{H}4). In humans, the isotypes are IgA, IgD, IgE, IgG, and IgM, with IgA and IgG further subdivided into subclasses or subtypes (IgA1-2 and IgG1-4).

Generally, variable domains show considerable amino acid sequence variability from one antibody to the next, particularly at the location of the antigen-binding site. Three regions, called hyper-variable or complementarity-determining regions (CDRs), are found in each of VL and VH, which are supported by less variable regions called framework variable regions. F11R/JAM-A antibodies may include IgG monoclonal antibodies as well as antibody fragments or engineered forms. These include, for example, Fv fragments, or proteins where the CDRs and/or variable domains of the exemplified antibodies are engineered as single-chain antigen-binding proteins.

A portion of an antibody consisting of the VL and VH domains is designated as an Fv (Fragment variable) and constitutes the antigen-binding site. A single chain Fv (scFv or SCA) may include an antibody fragment containing a VL domain and a VH domain on one polypeptide chain, where the N terminus of one domain and the C terminus of the other domain are joined by a flexible linker. The peptide linkers used to produce the single chain antibodies are typically flexible peptides, selected to assure that the proper three-dimensional folding of the VL and VH domains occurs. A linker may be about 10 to 50 amino acid residues, and in some instances is shorter, e.g., about 10 to 30 amino acid residues, or 12 to 30 amino acid residues, or even 15 to 25 amino acid residues. An example of such linker peptides includes repeats of four glycine residues followed by a serine residue.

Single chain antibodies lack some or all of the constant domains of the whole antibodies from which they are derived. Therefore, single chain antibodies can overcome problems associated with the use of whole antibodies. For example, single-chain antibodies are generally free of certain undesired interactions between heavy-chain constant regions and other biological molecules. Moreover, single-chain antibodies are considerably smaller than whole antibodies and can have greater permeability than whole antibodies, allowing single-chain antibodies to localize and bind to target antigen-binding sites more efficiently. Furthermore, the relatively small size of single-chain antibodies makes them less likely to provoke an unwanted immune response in a recipient than whole antibodies.

Fab (Fragment, antigen binding) refers to the fragments of the antibody consisting of the VL, CL, VH, and C_{H}1 domains. Those generated following pepsin digestion are referred to as Fab and do not retain the heavy chain hinge region. Following pepsin digestion, various Fabs retaining the heavy chain hinge are generated. Those fragments with the interchain disulfide bonds intact are referred to as F(ab')₂, while a single Fab' results when the disulfide bonds are not retained. F(ab')₂ fragments have higher avidity for antigen than the monovalent Fab fragments.

Fc (Fragment crystallization) denotes the portion or fragment of an antibody that comprises paired heavy chain constant domains. In an IgG antibody, for example, the Fc comprises C_{H}2 and C_{H}3 domains. The Fc of an IgA or an IgM antibody further comprises a C_{H}4 domain. The Fc is associated with Fc receptor binding, activation of complement mediated cytotoxicity, and antibody-dependent cellular-cytotoxicity (ADCC). For antibodies such as IgA and IgM, which are complexes of multiple IgG-like proteins, complex formation requires Fc constant domains.

The hinge region separates the Fab and Fc portions of the antibody, which allows for mobility of Fabs relative to each other and relative to Fc, as well as including multiple disulfide bonds for covalent linkage of the two heavy chains.

Antibody specificity refers to selective recognition of the antibody for a particular epitope of an antigen. The term epitope as used herein includes any protein determinant capable of specifically binding to an immunoglobulin or T-cell receptor or otherwise interacting with a molecule. Epitopic determinants may include chemically active surface groupings of molecules like amino acids, carbohydrates, or sugar side chains and generally have specific three-dimensional structural characteristics, as well as specific charge characteristics. An epitope may be linear or conformational. In a linear epitope, all points of interaction between the protein and the interacting molecule (such as an antibody) occur linearly along the primary amino acid sequence of the protein. In a conformational epitope, points of interaction occur across amino acid residues on the protein that are separate from one another, i.e., noncontiguous amino acids juxtaposed by tertiary folding of a protein. Epitopes formed from contiguous amino acids usually remain after exposure to denaturing solvents, whereas epitopes formed by tertiary folding are typically lost upon denaturing solvent application. An epitope typically includes at least 3, and often more (e.g., at least 5 or 8-10) amino acids in a unique spatial conformation. Antibodies that recognize the same epitope can be verified in a simple immunoassay showing the ability of one antibody to block the binding of another antibody to a target antigen.

Monoclonal antibodies of the present disclosure may be murine, human, humanized or chimeric. A humanized antibody is a recombinant protein in which the CDRs of an antibody from one species; e.g., a rodent, rabbit, dog, goat, horse, or chicken antibody (or any other suitable animal antibody), are transferred into human heavy and light variable domains. The constant domains of the antibody molecule are derived from those of a human antibody. Methods for making humanized antibodies are known in the art. Chimeric antibodies preferably have constant regions derived substantially or exclusively from human antibody constant regions and variable regions derived substantially or exclusively from the sequence of the variable region from a mammal other than a human. The chimerization process can be made more effective by also replacing the variable regions-other than the hyper-variable regions or the complementarity-determining regions (CDRs), of a murine (or other non-human mammalian) antibody with the corresponding human sequences. The variable regions other than the CDRs are also known as the variable framework regions (FRs).

The above-described antibodies can be obtained in accordance with standard techniques. For example, an immunologically active fragment of F11R/JAM-A can be administered to a subject (e.g., a mammal such as a human or mouse). The antibodies can be used by themselves as immunogens or they can be attached to a carrier protein or other objects, such as sepharose beads. After the mammal has produced antibodies, a mixture of antibody producing cells, such as splenocytes, are isolated, from which polyclonal antibodies may be obtained. Monoclonal antibodies may be produced by isolating individual antibody-producing cells from the mixture and immortalizing them by, for example, fusing them with tumor cells, such as myeloma cells. The resulting hybridomas are preserved in culture and the monoclonal antibodies are harvested from the culture medium.

For purposes of this and other aspects of the disclosure, the target "subject" encompasses any animal, preferably a mammal, more preferably a human. In the context of administering a composition of the disclosure for purposes of treating neointimal hyperplasia in a subject, the target subject encompasses any subject that is at risk of being infected by neointimal hyperplasia. Particularly susceptible subjects include adults and elderly adults. However, any infant, juvenile, adult, or elderly adult at risk for neointimal hyperplasia can be treated in accordance with the methods of the present disclosure. In the context of administering a composition of the disclosure for purposes of treating neointimal hyperplasia in a subject, the target subject population encompasses any subject having neointimal hyperplasia or at risk of having neointimal hyperplasia. In one embodiment, particularly suitable subjects include those at risk of infection or those infected with chronic kidney disease, end stage renal disease, or both. In one embodiment, the peptide administered is an amino acid sequence of SEQ ID NO: 3 and the condition is selected from the group consisting of chronic kidney disease and end stage renal disease. In another embodiment, suitable subjects include those having a blood vessel abnormality such as an arterio-venous fistula and/or an arterio-venous graft. In one embodiment, the peptide administered is an amino acid sequence of SEQ ID NO: 3 and the blood vessel abnormality is arterio-venous fistula or an arterio-venous graft.

In yet another embodiment, particularly suitable subjects include those who have undergone a procedure such surgical revision or angioplasty. For example, a subject may be one who has undergone an endarterectomy. In one embodiment, the peptide administered is an amino acid sequence of SEQ ID NO: 3 and the subject has undergone a procedure including but not limited to surgical revision and angioplasty.

Suitable subjects, in another embodiment, include those that have undergone renal replacement therapy. In one embodiment, the renal replacement therapy is hemodialysis. Alternatively, the renal replacement therapy may include peritoneal dialysis, hemofiltration, hemodiafiltration, intestinal dialysis, or any combination thereof. In one embodiment, the peptide administered is an amino acid sequence of SEQ ID NO: 3 and the subject has undergone renal replacement therapy, for example, hemodialysis.

As used herein, the phrase "therapeutically effective amount" means an amount of active compound or pharmaceutical agent that elicits the biological or medicinal response that is being sought in a tissue, system, animal, individual or human by a researcher, veterinarian, medical doctor or other clinician. The therapeutic effect is dependent upon the disorder being treated or the biological effect desired. As such, the therapeutic effect can be a decrease in the severity of symptoms associated with the disorder and/or inhibition (partial or complete) of progression of the disorder, or improved treatment, healing, prevention or elimination of a disorder, or side-effects. The amount needed to elicit the therapeutic response can be determined based on the age, health, size and sex of the subject. Optimal amounts can also be determined based on monitoring of the subject's response to treatment. The term "treatment" or "treat" may include effective inhibition, suppression or cessation of the F11R/JAM-A activity so as to prevent or delay the onset, retard the progression, or ameliorate the symptoms of the disorder.

In the context of using compositions of the present disclosure to prevent neointimal hyperplasia, the concentration of F11R/JAM-A proteins or polypeptides or anti-F11R/JAM-A antibodies in the composition are adequate to achieve the prevention of neointimal hyperplasia, particularly the prevention of neointimal hyperplasia in susceptible populations. In the context of using compositions to treat neointimal hyperplasia, the amounts of F11R/JAM-A proteins or polypeptides or anti-F11R/JAM-A antibodies are capable of achieving a reduction in a number of symptoms, a decrease in the severity of at least one symptom, or a delay in the further progression of at least one symptom, or even a total alleviation of the neointimal hyperplasia.

As used herein, the term "simultaneous" therapeutic use refers to the administration of at least one additional agent beyond the F11R/JAM-A inhibitor, for example, agents administered before, during, or after hemodialysis, peritoneal dialysis, or kidney transplant, optionally, by the same route and at the same time or at substantially the same time. As used herein, the term "separate" therapeutic use refers to an administration of at least one additional agent beyond the F11R/JAM-A inhibitor, for example, agents administered before, during, or after hemodialysis, peritoneal dialysis, or kidney transplant, at the same time or at substantially the same time by different routes. As used herein, the term "sequential" therapeutic use refers to administration of at least one additional agent beyond the F11R/JAM-A inhibitor, for example, agents administered before, during, or after hemodialysis, peritoneal dialysis, or kidney transplant, at different times, the administration route being identical or different. More particularly, sequential use refers to the whole administration of the additional agent before administration of the F11R/JAM-A inhibitor. It is thus possible to administer the additional agent over several minutes, hours, or days before applying the F11R/JAM-A inhibitor. In a preferred embodiment, the composition is administered before, during, or after surgery, and optionally at the locus of a vein. For example, the composition may be administered after a subject undergoes an aterio-venous fistula or an aterio-venous graft procedure.

In one embodiment, the additional agent may be a nanoparticle, which includes but is not limited to any nanoparticle constructed with complex organic surface layers on a metal core such as gold or mineral core such as silica, as well as nanoparticles constructed with a polymeric organic core consisting of micelles, dendrimers, dextran, or PLGA. Nanoparticles are well known in the art.

The composition may optionally be administered by any of various medically known or accepted or approved means of applying or administering such beneficial compositions. For example, the composition, in one embodiment, may be administered by intravenous injection, by intra-arterial injection, by intramuscular injection, intraperitoneally, intraplurally, orally, by inhalation, by intranasal instillation, topically, transdermally, parenterally, subcutaneously, or by application to mucous membrane (such as that of the nose, throat, bronchial tubes, genitals, and/or anus). The composition may be administered intranasally or by aerosol inhalation. In one preferred embodiment, administering is carried out parentally. In some embodiments, the composition may be incorporated into pharmaceutical compositions suitable for administration, as described herein. Useful routes of administration are by intravenous or intra-arterial injection (such as via the pulmonary artery), or intramuscular injection. Another example of a useful route of administration is subcutaneous although others can be equally effective. Intramuscular injection is most typically performed in the arm or leg muscles. Intravenous injections as well as intraperitoneal injections, intra-arterial, intracranial, or intradermal injections are also effective in generating desired response. Additional modes of administration include, for example, intratracheal inoculation, aspiration, airway instillation, nebulization, intranasal instillation, oral or nasogastric instillation, intraperitoneal injection, intravascular injection, intraventricularly, intralesionally, or implantation of a sustained release vehicle. In a particular embodiment, the administering carried out by intravenous injection, intra-arterial injection, intramuscular injection, intraperitoneally, topically, transdermally, subcutaneously, orally, or any combination thereof. In one embodiment, preferred administration is by venous injection. Alternatively, administration may be completed by aterial injection.

The pharmaceutical forms suitable for injection include sterile aqueous solutions or dispersions and sterile powders for the extemporaneous preparation of sterile injectable solutions or dispersions. In all cases the ultimate solution form must be sterile and fluid. Typical carriers include a solvent or dispersion medium containing, for example, water buffered aqueous solutions (i.e., biocompatible buffers), ethanol, polyols such as glycerol, propylene glycol, polyethylene glycol, suitable mixtures thereof, surfactants or vegetable oils. Sterilization can be accomplished by any art-recognized technique, including but not limited to, filtration or addition of antibacterial or antifungal agents, for example, paraben, chlorobutanol, phenol, sorbic acid or thimerosal. Further, isotonic agents such as sugars or sodium chloride can be incorporated in the subject compositions. Production of sterile injectable solutions containing the subject peptides is accomplished by incorporated these compounds in the required amount in the appropriate solvent with various ingredients enumerated above, as required, followed by sterilization, preferably filter sterilization. To obtain a sterile powder, the above solutions are vacuum-dried or freeze-dried as necessary.

Formulations suitable for injectable use include sterile aqueous solutions or dispersions and sterile powders for the extemporaneous preparation of sterile injectable solutions or dispersions. The form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils. As used herein, a pharmaceutically acceptable carrier may include any and all coatings, antibacterial and antifungal agents, isotonic agents the like. The use of such media and agents are well-known in the art. The pharmaceutically acceptable carriers used in conjunction with the peptides of the present disclosure vary according to the mode of administration. For example, the compositions can be formulated in any suitable carrier for oral liquid formulation such as suspensions, elixirs and solutions. Compositions for liquid oral dosage include any of the usual pharmaceutical media such as, for example, water, oils, alcohols, flavoring agents, preservatives, coloring agents and the like. In the case of oral solid preparations (capsules and tablets) carriers such as starches, sugars, diluents, granulating agents, lubricants, binders, disintegrating agents and the like can be used. In addition, carriers such as liposomes and microemulsions can be used.

When the compositions of the disclosure are administered orally, the compositions containing an effective dose of the F11R/JAM-A inhibitor can also contain an inert diluent, as assimilable edible carrier and the like, be in hard or soft shell gelatin capsules, be compressed into tablets, or can be in an elixir, suspension, syrup or the like.

The compounds of the present disclosure may be formulated for parenteral administration. Solutions or suspensions of the agent can be prepared in water suitably mixed with a surfactant such as hydroxypropylcellulose. Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil. In general, water, saline, aqueous dextrose and related sugar solution, and glycols, such as propylene glycol or polyethylene glycol, are preferred liquid carriers, particularly for injectable solutions. Under ordinary conditions of storage and use, these preparations contain a preservative to prevent the growth of microorganisms.

Compositions containing the F11R/JAM-A inhibitor of the present disclosure may be administered intravenously to inhibit, suppress, or cause the cessation of at least one F11R/JAM-A-mediated biological activity. When administered intravenously, the peptide compositions can be combined with other ingredients, such as carriers and/or adjuvants. The peptides may also be covalently attached to a protein carrier, such as albumin, to minimize clearing of the peptides. There are no limitations on the nature of the other ingredients, except that such ingredients must be pharmaceutically acceptable, efficacious for their intended administration and cannot degrade the activity of the active ingredients of the compositions. Examples of other anti-inflammatory ingredients contemplated by the present disclosure include, but are not limited to anti-F11R antibodies, NSAIDS, steroids, or cyclosporin-A. When employed together with F11R/JAM-A antagonists, these agents may be employed in lesser dosages than when used alone.

When it is desirable to deliver the agents of the present disclosure systemically, they may be formulated for parenteral administration by injection, e.g., by continuous infusion or bolus injection. Formulations for injection may be presented in unit dosage form, for example, in ampoules or in multi-dose containers with an added preservative. The compositions may take the form of suspensions, solutions, or emulsions in oily or aqueous vehicles, and may contain formulatory agents such as suspending, stabilizing, and/or dispersing agents.

Intraperitoneal or intrathecal administration of the agents may also be achieved using infusion pump devices, which allow continuous infusion of desired compounds avoiding multiple injections and multiple manipulations.

The agents may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

Compositions of the present disclosure may be administered in a single dose, or in accordance with a multi-dosing protocol. For example, relatively few doses of the therapeutic composition are administered, such as one or two doses. In embodiments that include conventional antibiotic therapy, which generally involves multiple doses over a period of days or weeks, the antibiotics can be taken one, two or three or more times daily for a period of time, such as for at least 5 days, 10 days or even 14 or more days, while the antibody composition is usually administered only once or twice. However, the different dosages, timing of dosages and relative amounts of the composition can be selected and adjusted by one of ordinary skill in the art.

The amount to be administered will, of course, vary depending upon the treatment regimen. Generally, an agent is administered to achieve an amount effective for cell differentiation or stimulation, or treatment of the condition causing or making a subject susceptible to having reduced differentiation or stimulation of cells. Thus, a therapeutically effective amount can be an amount which is capable of at least partially treating or preventing such a condition. This includes, without limitation, delaying the onset of infection. The dose required to obtain an effective amount may vary depending on the agent, formulation, and individual to whom the agent is administered.

Dosage, toxicity and therapeutic efficacy of the agents or compositions that inhibit F11R/JAM-A can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices may be desirable. While compositions that exhibit toxic side effects may be used, care should be taken to design a delivery system that targets such compositions to the site of affected tissue in order to minimize potential damage to uninfected cells and, thereby, reduce side effects.

Therapeutically effective amounts take numerous factors into account, including, for example, the concentrations of these active agents in the composition, the mode and frequency of administration, the severity of the neointimal hyperplasia to be treated (or prevented), and subject details, such as age, weight and overall health and immune condition. General guidance can be found, for example, in the publications of the International Conference on Harmonization and in REMINGTON'S PHARMACEUTICAL SCIENCES (Mack Publishing Company 1990), which is hereby incorporated by reference in its entirety. A clinician may administer F11R/JAM-A inhibitors, F11R/JAM-A peptides, or anti-F11R/JAM-A antibodies, until a dosage is reached that provides the desired or required prophylactic or therapeutic effect. The progress of this therapy can be easily monitored by conventional assays. The present disclosure also contemplates that the compositions can be suitably coated on stents, lines, and tubes with a therapeutically effective amount of the inhibitor or peptide which amount can be readily determined by the skilled practitioner.

Therapeutically effective amounts of F11R/JAM-A inhibitors will depend on whether an adjuvant or other agent is co-administered, with higher dosages being required in the absence of adjuvant. The amount of F11R/JAM-A inhibitors for administration sometimes varies from between about 1µg to about 1 g per dose and more usually from 5 µg to 500 mg per injection for human administration. For example, a dose of 50 mg may be used for a single human injection. The compositions of this disclosure may eventually be cleared from the bloodstream; thus, re-administration of the composition is indicated. The timing of injections can vary significantly from once a day, to once a year, to once a decade. Generally an effective dosage can be monitored by obtaining a fluid sample from the subject, generally a blood serum sample, and determining the titer of antibody developed against the F11R/JAM-A protein or polypeptide, using methods well known in the art and readily adaptable to the specific antigen to be measured. Ideally, a sample is taken prior to initial dosing and subsequent samples are taken and tittered after each administration. Generally, a dose or dosing schedule which provides a detectable titer at least four times greater than control or "background" levels at a serum dilution of 1:100 is desirable, where background is defined relative to a control serum or relative to a plate background in ELISA assays.

Particular embodiments of the invention include:
1. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A).
2. The method of embodiment 1, wherein said blood vessel abnormality is an arterio-venous fistula or an arterio-venous graft.
3. The method of embodiment 1, wherein said subject has a condition, and said condition is selected from the group consisting of chronic kidney disease and end stage renal disease.
4. The method of embodiment 1, wherein said subject has undergone a procedure, and said procedure is selected from the group consisting of surgical revision and angioplasty.
5. The method of embodiment 1, wherein said subject has undergone renal replacement therapy.
6. The method of embodiment 5, wherein said renal replacement therapy is hemodialysis.
7. The method of any one of embodiments 1 through 6, wherein said composition comprises a peptide and the peptide is selected from any one or more of an amino acid sequence of SEQ ID NO: 2, an amino acid sequence of SEQ ID NO: 3, an amino acid sequence of SEQ ID NO: 4, an amino acid sequence of SEQ ID NO: 5, an amino acid sequence of SEQ ID NO: 6, an amino acid sequence of SEQ ID NO: 7, an amino acid sequence of SEQ ID NO: 8, an amino acid sequence of SEQ ID NO: 9, an amino acid sequence of SEQ ID NO: 11, an amino acid sequence of SEQ ID NO: 12, an amino acid sequence of SEQ ID NO: 13, an amino acid sequence of SEQ ID NO: 14, an amino acid sequence of SEQ ID NO: 16, and an amino acid sequence of SEQ ID NO: 17.
8. The method of embodiment 7, wherein said peptide is an amino acid sequence of SEQ ID NO: 3.
9. The method of embodiment 8, wherein said blood vessel abnormality is an arterio-venous fistula or an arterio-venous graft.
10. The method of embodiment 8, wherein said subject has a condition, and said condition is selected from the group consisting of chronic kidney disease and end stage renal disease.
11. The method of embodiment 8, wherein said subject has undergone a procedure, and said procedure is selected from the group consisting of surgical revision and angioplasty.
12. The method of embodiment 8, wherein said subject has undergone renal replacement therapy.
13. The method of embodiment 12, wherein said renal replacement therapy is hemodialysis.
14. The method of any one of embodiments 1 through 6, wherein said composition further comprises a pharmaceutically acceptable excipient.
15. The method of any one of embodiments 1 through 6, wherein said administering is carried out parentally.
16. The method of any of embodiments 1 through 6, wherein said administering carried out by intravenous injection, intra-arterial injection, intramuscular injection, intraperitoneally, topically, transdermally, subcutaneously, orally, or any combination thereof.
17. The method of any one of embodiments 1 through 6 further comprising:
   repeating said administering.
18. The method of embodiment 1, wherein the subject is a patient on hemodialysis.
19. The method of embodiment 1, wherein said composition further comprises a polyethylene glycol tag.
20. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 1.
21. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 2.
22. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 3.
23. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 4.
24. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 5.
25. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 6.
26. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 7.
27. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 8.
28. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 9.
29. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 10.
30. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 11.
31. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 12.
32. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 13.
33. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 14.
34. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 15.
35. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 16.
36. A method of treating neointimal hyperplasia, comprising:
   selecting a subject having a blood vessel abnormality, and
   administering to said subject an amount of a composition, wherein said composition comprises an inhibitor of cell adhesion molecule receptor/Junctional Adhesion Molecule-A (F11R/JAM-A), wherein said composition comprises a peptide and the peptide comprises an amino acid sequence of SEQ ID NO: 17.
37. The method of any one of embodiments 20 through 36, wherein said blood vessel abnormality is an arterio-venous fistula or an arterio-venous graft.
38. The method of any one of embodiments 20 through 36, wherein said subject has a condition, and said condition is selected from the group consisting of chronic kidney disease and end stage renal disease.
39. The method of any one of embodiments 20 through 36, wherein said subject has undergone a procedure, and said procedure is selected from the group consisting of surgical revision and angioplasty.
40. The method of any one of embodiments 20 through 36, wherein said subject has undergone renal replacement therapy.
41. The method of embodiment 40, wherein said renal replacement therapy is hemodialysis.
42. The method of any one of embodiments 20 through 36, wherein said composition further comprises a pharmaceutically acceptable excipient.
43. The method of any one of embodiments 20 through 36, wherein said administering is carried out parentally.
44. The method of any one of embodiments 20 through 36, wherein said administering carried out by intravenous injection, intra-arterial injection, intramuscular injection, intraperitoneally, topically, transdermally, subcutaneously, orally, or any combination thereof.
45. The method of any one of embodiments 20 through 36, further comprising:
   repeating said administering.
46. The method of any one of embodiments 20 through 36, wherein the subject is a patient on hemodialysis.
47. The method of any one of embodiments 20 through 36, wherein said composition further comprises a polyethylene glycol tag.

The present disclosure may be further illustrated by reference to the following examples.

### EXAMPLES

### Example 1 - Materials and Methods.

Carotid artery ligation was performed on 14 uremic C57BL/6 mice that underwent 5/6 nephrectomy. One week after nephrectomy, partial ligation of the left carotid artery (LCA) was performed on each mouse. Focal LCA stenosis was created by placing 9-0 nylon suture at 1mm proximal to the carotid bifurcation (mid-focal stenosis) around the artery using a 35-gauge blunt needle (World precision Instruments, Inc., Sarasota, FL). Then the needle mandrel was gently removed to restore blood flow.

Mice were fed a regular mouse diet and divided into 2 groups of 7 mice each; the experimental group received Peptide 4D while the Control group received saline injections by intraperitoneal route for 21 days, as this was the time shown in earlier studies to correlate with development of neointimal hyperplasia following partial ligation of the carotid artery.

The mice were then euthanized on day 21 and both LCA and right carotid artery (RCA) were excised for analysis. Specimens were embedded in paraffin. Serial sections (6 µm thick) were collected at 200-µm intervals of the LCA from mid-focal stenosis as well as from the RCA serving as lesion control. Data were analyzed qualitatively and quantitatively using Image J software. The two groups were compared for differences in mean Intima Area and Intima Index, defined as the ratio of intima area divided by intima plus media area.

Resulting from this example, no neointimal hyperplasia developed in the contralateral RCA in each group. All LCA in control mice developed severe neointimal hyperplasia with two having complete luminal occlusion. In experimental mice LCA, only one had severe neointimal hyperplasia and the rest of the 6 had significantly less neointimal hyperplasia compared with Control. Overall the mean Intima Index was significantly less in the experimental LCA group, compared with control group (0.31 + 0.14 vs. (0.47 + 0.15); p = 0.039. These results are illustrated in FIG. 6.

### Example 2 - Peptide 4D Significantly Inhibits Neointimal Hyperplasia.

Administration of Peptide 4D (having SEQ ID NO: 3) significantly inhibits neointimal hyperplasia in uremic C57BL/6 mice in a carotid ligation model.

Photographs of samples of carotid segments of the two groups, experimental group (mice treated with peptide 4D) and control group (mice injected with saline) were compared for differences in mean intimal, medial, lumen area, and intima Index (defined as the ratio of intima area divided by intima plus media area) are shown in FIGs. 1A-1B, 3A-3B, and 6.

FIGs. 2A-2C are representative Masson's trichrome-stained images of mid focal stenosis in uremic C57BL/6J mice. Mid-focal stenosis of a left carotid artery (LCA) in a uremic mouse injected with saline for 21 days is shown in FIG. 2A and Mid-focal stenosis of a LCA in a uremic mouse injected with Peptide 4D for 21 days is shown in FIG. 2B. FIG. 2C shows contralateral control of right carotid artery (RCA). The specimens were analyzed at three weeks after surgery. The scale bar in each image is equal to 100µm.

FIGs. 3A and 3B are graphical illustrations of mid focal stenosis morphometry of control uremic mice and uremic mice treated with peptide 4D. Morphometric analysis was performed at 21 days after left carotid artery mid focal stenosis. FIG. 3A is the control group of uremic mice injected with saline for 21 days. FIG. 3B is the group of uremic mice injected with peptide 4D for 21 days.

Each lane represents average of area relative to distance the focal stenosis to the proximal (Mean + SEM) of 10 location from each group of mice (n=7). Contralateral intimal area from right carotid artery had values zero.

FIG. 4 is graphical illustrations of the comparison of mid focal stenosis morphometry in left carotid artery of uremic mice treated with peptide 4D versus control untreated uremic mice.

In FIG. 4, the blue line represents control uremic mice injected with saline for 21 days, and the red line represents uremic mice treated with peptide 4D for 21 days. Each lane represents average of area relative to distance the focal stenosis to the proximal (Mean + SEM) of 10 location from each group of mice (n=7).

FIG. 4 is a comparison of intimal area of the comparison of mid focal stenosis morphometry in left carotid artery of uremic mice treated with peptide 4D versus control untreated uremic mice, showing that intima area was significantly smaller in treated mice than in control mice at any distance from the ligation point.

FIG. 5 is a graphical illustration of the comparison of the average of intimal area as a sum of 10 LCA locations with distal focal stenosis of untreated control uremic mice with uremic mice treated with peptide 4D (n=7). Overall the mean intimal area was significantly less in the experimental group (Peptide 4D) compared with control group (0.0082 + 0.0103) vs. (0.031 + 0.024). *Significance by Test U-Mann Whitney, p=0.011.

FIG. 6 is a graphical illustration of the comparison of the average of ratio of intima area/intima + media area (Intima Index) from 10 LCA locations with distal focal stenosis of untreated control uremic mice with uremic mice treated with peptide 4D (n=7). Overall the mean Intima Index was significantly less in the experimental LCA group, compared with control group (0.089 + 0.081) vs. (0.27 + 0.13); *Significance by Test U-Mann Whitney, p = 0.0079.

FIG. 7 is a graphical illustration of the comparison of the average of ratio of intima area/intima +media area (Intima Index) from 1 LCA location with biggest distal focal stenosis of untreated control uremic mice with uremic mice treated with peptide 4D (n=7). Overall the mean Intima Index was significantly less in the experimental LCA group, compared with control group (0.31 + 0.14) vs. (0.47 + 0.15); *Significance by Test U-Mann Whitney p = 0.039.

This example demonstrates the effect of F11R/JAM-A antagonist, peptide 4D on neointimal hyperplasia in mouse carotid ligation model. Focal left carotid artery (LCA) stenosis was created in uremic C57BL/6 mice and administration of Peptide 4D was performed for 21 days.

Thus, it was determined that 1) all LCA in control mice injected with saline developed severe neointimal hyperplasia with two having complete luminal occlusion; 2) In experimental mice LCA, only one had severe neointimal hyperplasia and the rest of the 6 had significantly less neointimal hyperplasia compared with control; and 3) No neointimal hyperplasia developed in the contralateral RCA in each group.

Accordingly, administration of Peptide 4D significantly inhibits neointimal hyperplasia in uremic C57BL/6 mice in a carotid ligation model.

While the novel technology has been illustrated and described in detail in the figures and foregoing description, the same is to be considered as illustrative and not restrictive in character, it being understood that only the preferred embodiments have been shown and described and that all changes and modifications that come within the spirit of the novel technology are desired to be protected. As well, while the novel technology was illustrated using specific examples, theoretical arguments, accounts, and illustrations, these illustrations and the accompanying discussion should by no means be interpreted as limiting the technology. All patents, patent applications, and references to texts, scientific treatises, publications, and the like referenced in this application are incorporated herein by reference in their entirety.

Although preferred embodiments have been depicted and described in detail herein, it will be apparent to those skilled in the relevant art that various modifications, additions, substitutions, and the like can be made without departing from the spirit of the disclosure and these are therefore considered to be within the scope of the disclosure as defined in the claims which follow.

## Claims

1. A composition for use in treating neointimal hyperplasia in a subject, the composition comprising an inhibitor of cell adhesion molecule receptor/Junctional Adhesion Molecule-A (F11R/JAM-A), wherein the subject has an arterio-venous fistula or an arterio-venous graft and has undergone renal replacement therapy.

2. The composition for use of claim 1, wherein the inhibitor comprises a peptide, wherein the peptide comprises an amino acid sequence selected from any one or more of SEQ ID NOs: 1-17, or an amino acid sequence having about 70-99% sequence similarity to any of SEQ ID NOs: 1-17.

3. The composition for use of claim 2, wherein the peptide comprises an amino acid sequence selected from any one or more of SEQ ID NOs: 2-17.

4. The composition for use of claim 3, wherein said amino acid sequence is SEQ ID NO: 3.

5. The composition for use of any one of claims 1-4, wherein said subject has chronic kidney disease or end stage renal disease.

6. The composition for use of claim 5, wherein said subject has chronic kidney disease.

7. The composition for use of claim 5, wherein said subject has end stage renal disease.

8. The composition for use of any one of claims 1-4, wherein said subject has undergone surgical revision or angioplasty.

9. The composition for use of claim 8, wherein said subject has undergone angioplasty.

10. The composition for use of claim 8, wherein said subject has undergone surgical revision.

11. The composition for use of any one of claims 1-4, wherein said renal replacement therapy is hemodialysis.

12. The composition for use of any one of claims 1-11, wherein said composition further comprises a pharmaceutically acceptable excipient.

13. The composition for use of any one of claims 1-11, wherein said composition is administered parentally.

14. The composition for use of any one of claims 1-11, wherein said composition is administered by intravenous injection, intra-arterial injection, intramuscular injection, intraperitoneally, topically, transdermally, subcutaneously, orally, intravascular local delivery via devices or any combination thereof.

15. The composition for use of any one of claims 1-11, wherein said composition is administered by:
(a) intravascular injection; or
(b) an infusion pump device.
